(19) Europäisches Patentamt
European Patent Office
Office européen des brevets

(11) Publication number: **0 226 730 B1**

(12) **EUROPEAN PATENT SPECIFICATION**

(45) Date of publication of patent specification: **02.03.94**

(51) Int. Cl.[5]: **C07D 223/28**, C07C 211/60, C07D 405/12, C07D 311/82, G01N 33/94, G01N 21/64, //C07D223/22,G01N33/533

(21) Application number: **86113654.7**

(22) Date of filing: **03.10.86**

The file contains technical information submitted after the application was filed and not included in this specification

(54) **Compounds and assay for tricyclic antidepressants.**

(30) Priority: **15.10.85 US 787359**

(43) Date of publication of application:
**01.07.87 Bulletin 87/27**

(45) Publication of the grant of the patent:
**02.03.94 Bulletin 94/09**

(84) Designated Contracting States:
**BE DE FR IT**

(56) References cited:

| | |
|---|---|
| **EP-A- 0 050 684** | **EP-A- 0 108 399** |
| **EP-A- 0 108 403** | **BE-A- 633 316** |
| **DE-A- 2 335 943** | **DE-A- 3 205 506** |
| **DE-C- 1 156 413** | **DE-C- 1 189 550** |
| **US-A- 4 070 373** | **US-A- 4 668 640** |

**CHEMICAL ABSTRACTS, vol. 78, no. 23, 11th June 1973, Columbus, Ohio, US, page 354, column 2, abstract no. 147657p; F.J. MARSHALL et al.: "Use of diborane and denteriodiborane in the synthesis of isotopically labelled amines";**

(73) Proprietor: **ABBOTT LABORATORIES**
**One Abbott Park Road**
**Abbott Park, Illinois 60064-3500(US)**

(72) Inventor: **Walters, Roland Lawrence**
**724 Concord Lane**
**Barrington Illinois 60010(US)**
Inventor: **Raden, Daniel S.**
**13 John Drive**
**Hawthorn Woods Illinois 60047(US)**
Inventor: **Hu, Hsiang-Yung**
**1763 Gedar Gren Drive**
**Libertyville Illinois 60048(US)**

(74) Representative: **Modiano, Guido, Dr.-Ing. et al**
**Modiano & Associati S.r.l.**
**Via Meravigli, 16**
**I-20123 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

**JOURNAL OF CHROMATOGRAPHY, vol. 204, 1981, pages 319-327; J. FEKETE et al.: "Reversed-phase liquid chromatography for the separation of chlorpromazine, imipramine and some of their metabolites"**

**JOURNAL OF CHROMATOGRAPHY, vol. 143, 1977, pages 499-512; J.C. KRAAK et al.: "Determination of amitriptyline and some of its metabolites in blood by high-pressure liquid chromatography"**

## Description

### BACKGROUND OF THE INVENTION

#### Technical Field

This invention relates to reagents and a method utilizing the reagents, for determining the presence or amount of tricyclic antidepressants, especially Imipramine, Desipramine, Amitriptyline, and Nortriptyline, in fluids, especially biological fluids such as serum, plasma, spinal and amniotic fluids and the like, and to a method for making the reagents. More particularly, the invention relates to haptens and immunogens used to raise antisera, and to tracers; the haptens, immunogens and tracers are useful as reagents in a fluorescence polarization immunoassay procedure to determine tricyclic antidepressants.

#### Background Art

The tricyclic antidepressants are a class of compounds which are used to treat chronic depression. The class is characterized by the general structure:

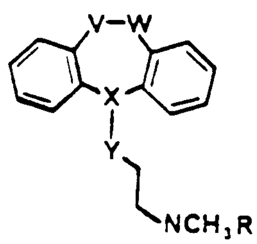

Where: V=$CH_2$ or HC=
W=$CH_2$, 0 or =CH
X=N or C=
Y=$CH_2$ or =CH
R=H or $CH_3$

Although these compounds have been found to be very effective in treating chronic depression, their concentrations in a patient's blood must be maintained in a therapeutic range. A wide interpatient variation normally exists in the steady-state concentration in human plasma, for a given dose. High doses have been associated with central nervous system disorders, cardiac toxicity, hypertension, seizures, coma and death. Since individuals vary greatly in their response, it is essential to monitor the therapy by measuring the serum or plasma levels of these drugs.

Typically, competitive binding immunoassays are used for measuring ligands in a test sample. (For the purposes of this disclosure, a "ligand" is a substance of biological interest to be determined by a competitive binding immunoassay technique) The ligands compete with a labeled reagent, or "ligand analog", or "tracer", for a limited number of receptor binding sites on antibodies specific to the ligand and ligand analog. The concentration of ligand in the sample determines the amount of ligand analog which binds to the antibody: the amount of ligand analog that will bind is inversely proportional to the concentration of ligand in the sample, because the ligand and the ligand analog each bind to the antibody in proportion to their respective concentrations.

In the past, patient serum/plasma levels of tricyclic antidepressants were measured by gas chromatography (GC), liquid chromatography (LC), or enzyme multiplied immunoassay. However, such assay methods are not without drawbacks. Both GC and LC are labor intensive, requiring highly skilled personnel to do extractions of the analyte from the biological matrix. Enzyme multiplied immunoassays are less labor intensive but are subject to variability due to temperature and matrix effects which alter enzyme activity.

Known from Journal of Chromatography, vol. 143, 1977, pages 499-512 is a method for the determination of amitriptyline and some of its metabolites in blood by high pressure liquid chromatography (HPLC).

Known from Journal of Chromatography, vol. 204, 1981, pages 319-327 is a method for the separation of chlorpromazine, imipramine and some of their metabolites by reversed-phase liquid chromatography.

Fluorescence polarization immunoassay procedures provide a quantitative means for measuring the amount of tracer-antibody conjugate produced in a competitive binding immunoassay. Such fluorescence polarization techniques are based on the principle that a fluorescent labeled compound, when excited by plane polarized light, will emit fluorescence having a degree of polarization inversely related to its rate of rotation. Accordingly, when a tracer-antibody conjugate having a fluorescent label is excited with plane polarized light, the emitted light remains highly polarized because the fluorophore is constrained from rotating between the time that light is absorbed and emitted. In contrast, when an unbound tracer is excited

by plane polarized light, its rotation is much faster than the corresponding tracer-antibody conjugate and the molecules are more randomly oriented. As a result, the light emitted from the unbound tracer molecules is depolarized. Such fluorescence polarization techniques are disclosed in U.S. Patent No. 4,420,568 to Wang et al., which is directed to the use of a triazinylaminofluorescein moiety as the fluorophore (tracer).

Known from US-A-4 668 640 to Wang et al. is a fluorescence polarization-based method for determining ligands in a sample utilizing a salt of a tracer consisting of a ligand-analog having a single reactive primary or secondary amino group which is attached to the carbonyl carbon of carboxy fluorescein wherein said ligand-analog has at least one common epitope with said ligand so as to be specifically recognizable by a common antibody.

In general, the class of ligand-analogs of US-A-4 668 640 are derived from the corresponding ligand by removal of a reactive hydrogen atom, i.e. a hydrogen atom bonded to a reactive amine (primary or secondary) or by the formation of an amino derivative of the ligand wherein an imino group - NH- replaces one or more atoms originally present in the ligand, at the site of binding to the carboxyfluorescein moiety.

Representative ligands determinable by the method of US-A-4 668 640 include, among many others, antidepressant drugs including tricyclics such as nortriptyline, amitriptyline, imipramine and desipramine and the like as well as the metabolites thereof. Specifically disclosed are desipramine and nortriptyline, among others, as illustrative of such ligands which upon removal of a reactive hydrogen may form ligand-analogs in the preparation of a tracer.

It may be mentioned that some of the compounds used according to the present invention have been previously disclosed. In this regard, mention is made firstly of DE-C-1 156 413 and DE-C-1 189 550, which relate to methods for preparing azepine derivatives. US-A-4 070 373 relates to tricyclic aminoalkyl derivatives for pharmaceutical use. BE-A-633 316 deals with a method for the preparation of dibenzocycloheptenes. Journal of Labelled Compounds, vol. VIII, no. 3 (1972), p. 461-473 deals with the synthesis of isotopically labeled amines.

## SUMMARY OF THE INVENTION

The present invention offers an advance in the art beyond the Wang, et al. patent previously described, particularly in that highly sensitive fluorophores, a method for making the fluorophores, and an assay using the fluorophores as tracers are provided specifically for the determination of one or more tricyclic antidepressants with one assay. An assay conducted in accordance with the present invention is particularly accurate, as will be explained infra.

This invention is directed to haptens and immunogens used to raise antibody which can be used in an assay for tricyclic antidepressants, to tracers for use in fluorescence polarization immunoassays for tricyclic antidepressants, to methods for conducting such assays, and to methods for making the tracers, haptens and immunogens.

Accordingly, a first aspect of the invention relates to the discovery that certain compounds which, when covalently attached to protein and used to raise antibodies, provide antisera with high cross reactivities for a number of different tricyclic antidepressants. Thus, according to the first aspect of the present invention, such compounds used to raise antibodies are 5-substituted 10,11-dihydro-5H-dibenzo(b,f)azepine (compounds of Formula A), and 5-substituted 10,11-dihydro-5H-dibenzo(a,d)cycloheptene (compounds of Formula B):

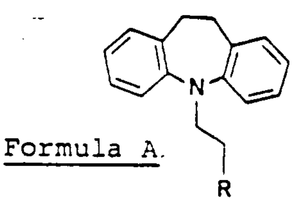

Formula A.

where R=$NH_2$ or $NHCH_3$

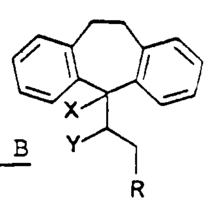

Formula B

Where R =$(CH_2)_nNH_2$ or $(CH_2)_nNHCH_3$; and when X=Y=H, then n=0 or 1; when X and Y form a bond, then n=0

A second aspect of the invention relates to the discovery of unique fluorophore tracer compounds having novel structures. Thus, according to the second aspect of the invention, there are provided compounds which are conjugates of 5-substituted-10,11-dihydro-5H-dibenzo(a,d)cycloheptene (Formula B) with 5- or 6-substituted fluorescein (compounds of Formula C), and biologically acceptable salts thereof:

Formula C

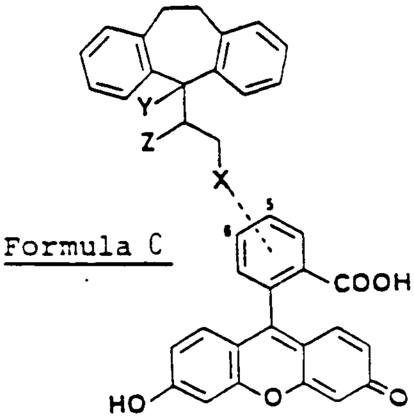

where: Y and Z are both H or together form a bond;

X is $-(CH_2)_n-NR-\overset{O}{\overset{\parallel}{C}}-$; n is 0 or 1 when Y and Z are H and zero when Y and Z form a bond;

R is H or $CH_3$

The dotted line in the foregoing structures indicates that the point of attachment to the fluorescein moiety can be at either the 5 or 6 position.

A third aspect of the invention relates to an analytical method, i.e., a method for conducting an assay using as reagents compounds of Formula C and antisera raised in response to immunogens prepared from haptens of Formula A or B. According to the third aspect of the invention, an improved fluorescence polarization immunoassay is provided, comprising the steps of contacting a fluid suspected of containing tricyclic antidepressant(s) with antisera to a tricyclic antidepressant and a fluorescent-labeled tricyclic derivative (e.g., a compound of Formula C ) capable of producing a detectable fluorescence polarization response to the presence of the antiserum in a homogeneous solution passing plane polarized light through the homogeneous solution, and measuring the fluorescence polarization response therefrom.

Further objects and attendant advantages of the invention will be apparent from the following detailed description taken together with the Examples.

## DETAILED DESCRIPTION OF THE INVENTION

The present invention is directed to haptens and immunogens (to raise antisera) and to tracers for use in an improved fluorescence polarization immunoassay for tricyclic antidepressants; to synthetic methods, for making the foregoing reagents; and to an analytical method, using the reagents, for detecting the presence or amount of tricyclic antidepressants in fluids, e.g., human body fluids including blood serum, plasma, spinal fluid and the like.

Compounds of Formula B can be prepared by treatment of the corresponding halides with the appropriate amine in alcohols, where R' = halogen, by well-known procedures, for example those described in U.S. Patents 3,981,917 and 3,978,121, the disclosures of which are hereby incorporated by reference. The procedure set forth in Example 4, infra, has also been used to prepare immunogens in accordance with the invention.

Specifically, it has been found in accordance with the invention that immunogens prepared from haptens of the structures of Formula A or Formula B, when used to raise antisera in animals using techniques well known to those skilled in the art, will yield antisera with unexpected advantageously high cross reactivities to the major tricyclic antidepressants but with low cross reactivities to their major metabolites and other commonly used antidepressants. The relative responses of the preferred antisera to tricyclic antidepressants and their metabolities are shown in Table I. The cross reactivities are relative to imipramine which is taken as 100% at each concentration.

The cross reactivity was established by spiking pooled normal human serum with the drugs to be tested up to 1 microgram per milliliter (mcg/ml), and then assaying the samples in the TDx$^{R}$ TCAs system, a commercially available instrumental analysis system utilizing fluorescence polarization techniques (from Abbott Laboratories, Abbott Park, Illinois).

TABLE I

| COMPOUND TESTED | SERUM CONCENTRATION (nanograms per milliliter) | % CROSS REACTIVITY |
|---|---|---|
| A. Major Tricyclics | | |
| Desipramine | 100 | 99 |
| | 300 | 94 |
| | 500 | 92 |
| Amitriptyline | 100 | 101 |
| | 300 | 103 |
| | 500 | 109 |
| Nortriptyline | 100 | 95 |
| | 300 | 90 |
| | 500 | 86 |
| B. Minor Tricyclics | | |
| Doxepin | 100 | 58 |
| | 300 | 45 |
| | 500 | 39 |
| Trimipramine | 100 | 93 |
| | 300 | 74 |
| | 500 | 70 |
| Clomipramine | 100 | 51 |
| | 300 | 46 |
| | 500 | 41 |
| Protriptyline | 100 | 113 |
| | 300 | 102 |
| | 500 | 96 |
| C. Metabolites | | |
| Imipramine N-Oxide | 1000 | 0.7 |
| 2-Hydroxyimipramine | 300 | 18 |
| | 1000 | 11 |
| 2-Hydroxy desipramine | 300 | 8 |
| | 1000 | 13 |
| 10-Hydroxyamitriptyline-CIS | 300 | 9 |
| TRANS | 300 | 6 |
| 10-Hydroxynortriptyline-CIS | 300 | 6 |
| TRANS | 300 | 6 |
| D. Other Antidepressants | | |
| Maprotiline | 1000 | 14 |
| Amoxapine | 1000 | 2 |
| Trazodone | 1000 | 1 |

As previously stated, the tracers of the present invention can be of the structure of Formula C and biologically acceptable salts thereof. Most preferred are the compounds of Formula C in which Y=Z=H, due to the greater intensity of the fluorescence response produced in these structures which is particularly advantageous in the practice of the method of the present invention to measure the presence or amount of tricyclic antidepressants in biological fluids.

The tracers of the present invention generally exist in an equilibrium between their acid and ionized states, and in the ionized state are effective in the method of the present invention. Therefore, the present invention comprises the tracers in either the acid or ionised state, and for convenience, the tracers of the present invention are structurally represented herein in their acid form. When the tracers of the present invention are present in their ionized state, the tracers exist in the form of biologically acceptable salts. As used herein, the term "biologically acceptable salts" refers to salts such as sodium potassium, ammonium and the like which will enable the tracers of the present invention to exist in their ionized state when employed in the method of the present invention. Generally, the tracers of the present invention exist in solution as salts, with the specific salt resulting from the buffer employed. For example, in the presence of a

sodium phosphate buffer, the tracers of the present invention will generally exist in their ionized state as a sodium salt.

A tracer which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule which is slower than that of the relatively small tracer molecule, thereby increasing the polarization observed. Therefore, when a ligand competes with the tracer for antibody sites, the observed polarization of fluorescence of the tracer-antibody complex becomes a value somewhere between that of the tracer and tracer-antibody complex. If a sample contains a high concentration of the ligand, the observed polarization value is closer to that of the free ligand; i.e., low. If the test sample contains a low concentration of the ligand, the polarization value is closer to that of the bound ligand; i.e., high. By sequentially exciting the reaction mixture of an immunoassay with vertically and then horizontally polarized light and analyzing only the vertical component of the emitted light, the polarization of fluorescence in the reaction mixture may be accurately determined. The precise relationship between polarization and concentration of the ligand to be determined can be established by measuring the polarization values of calibrators with known concentrations. The concentration of the ligand can be extrapolated from a standard curve prepared in this manner.

The results can be quantified in terms of net millipolarization units, span (in millipolarization units) and intensity. The measurement of millipolarization units indicates the maximum polarization when a maximum amount of the tracer is bound to the antibody in the absence of any tricyclic antidepressant. The higher the net millipolarization, the better the binding of the tracer to the antibody. The span is an indication of the difference between the net millipolarization and the minimum amount of tracer bound to the antibody. A larger span provides for a better numerical analysis of data. The intensity is a measure of the strength of the signal above background. Thus, a higher intensity will give a more accurate measurement. The intensity is determined, at about a 2 nanomolar concentration of tracer, as the sum of the vertically polarized intensity plus twice the horizontally polarized intensity.

In accordance with a preferred method of conducting the assay of the present invention, a sample containing tricyclic antidepressant(s) or suspected of containing tricyclic antidepressant(s), is intermixed with a biologically acceptable salt of a tracer, such as the tracers illustrated in Formula C , and an antibody specific for one or more tricyclic antidepressants and the tracer. The tricyclic antidepressant(s) and the tracer compete for limited antibody sites, resulting in the formation of tricyclic antidepressant-antibody and tracer-antibody complexes. By maintaining constant the concentration of tracer and antibody, the ratio of tricyclic antidepressant-antibody complex to tracer-antibody complex that is formed is directly proportional to the amount of tricyclic antidepressant(s) present in the sample. Therefore, upon exciting the mixture with plane polarized light and measuring the polarization of the fluorescence emitted by the tracer and the tracer-antibody complex, one is able quantitatively to determine the amount of tricyclic antidepressant(s) in the sample.

The pH at which an assay of the present invention is carried out must be sufficient to allow the tricyclic antidepressant tracer utilized to exist in its ionized state. The pH may range from about 3 to 12, more usually in the range of from about 5 to 10, most preferably from about 6 to 9. Various buffers may be used to achieve and maintain the pH during the assay procedure. Representative well-known buffers include borate, phosphate, carbonate, tris, barbital and the like. The particular buffer employed is not critical to the present invention, but the tris and phosphate buffers are preferred. The cation portion of the buffer will generally determine the cation portion of the tracer salt in solution.

The reagents for the most preferred fluorescence polarization assay of the present invention comprise antibody specific for one or more tricyclic antidepressant(s) raised in response to haptens of Formula A, where $R = NH_2$, which have been conjugated to bovine serum albumin and a tracer of Formula C, where $Y = Z = H$ and $X = CH_2\text{-}CH_2\text{-}NCH_3\text{-}C = O$, with attachment to the fluorescein moiety being through the 6 position. Additionally, a pretreatment solution to extract the tricyclic antidepressant(s) being assayed, a dilution buffer, tricyclic antidepressant calibrators and tricyclic antidepressant controls are desirably prepared.

A preferred procedure for the determination of tricyclic antidepressants in accordance with the invention is set forth in the following discussion. All percentages expressed herein are weight/volume unless otherwise indicated.

The tracer formulation presently preferred is a concentration of 82 nanomolar tracer (e.g., compounds of Formula C , supra) in: 0.1 molar Tris buffer at pH 7.5; 2.0% sodium octyl sulfate; 0.1% sodium azide; and 0.01% bovine gamma globulin. The antiserum formulation comprises rabbit serum diluted with: 0.1 molar

phosphate buffer at pH 6.4; 0.1% sodium azide; 0.01% bovine gamma globulin; 1% human serum (volume/volume); and 2% ethylene glycol (volume/volume). The dilution buffer comprises: 0.1 molar sodium phosphate at pH 7.5; 0.1% sodium azide; and 0.01% bovine gamma globulin. The pretreatment formulation comprises: 0.01% bovine gamma globulin; 0.1 molar tris buffer at pH 7.5; 0.1% sodium azide; and 2.0% sodium octyl sulfate. Tricyclic antidepressant calibrators comprising imipramine and normal human serum at concentrations of 0, 75, 150, 300, 600 and 1000 nanograms per milliliter, with 0.1% sodium azide preservative, are useful and preferred. Tricyclic antidepressant controls comprising imipramine and normal human serum at concentrations of 100, 200 and 500 nanograms per milliliter, with 0.1% sodium azide as a preservative, are also useful and preferred.

The preferred procedure is especially designed to be automated and used in conjunction with the Abbott TDx$^R$ Fluorescence Polarization Analyzer, commercially available from Abbott Laboratories, Abbott Park, Illinois. In this procedure, fifty microliters of serum or plasma are required. The calibrators, controls or unknown samples are pipetted directly into the sample well of the TDx sample cartridge. One of the advantages of this procedure is that the sample does not require any special preparation. For example, if a TDx tricyclic antidepressant assay kit is being used with the TDx analyzer to perform assays in accordance with the present invention, the caps from each of the three reagent containers in the kit are removed and placed into designated wells inside the TDx Analyzer, and the assay procedure from this point is fully automated.

However, if a manual assay is being performed according to the invention, then the sample is preferably mixed with the pretreatment solution in dilution buffer, and a background reading is taken. The tracer is then mixed with the test solution, and antibody is thereafter mixed into the solution. After incubation, a fluorescence polarization reading is taken.

In instrumental determinations, the fluorescence polarization value of each calibrator, control or sample can be determined, and printed on the output tape of an instrument such as the Abbott TDx Analyzer. A standard curve is generated in the instrument, plotting the polarization of each calibrator versus its concentration using a nonlinear regression analysis. The concentration of each control or sample is read off the stored calibration curve and printed on the output tape.

Complete details and instructions on procedural aspects of the performance of automated assays using the compounds of the invention are available in the TDx Analyzer Systems Operation Manual, available from Abbott Laboratories, Abbott Park, Illinois.

With respect to the foregoing preferred procedure, it should be noted that the tracer, antibody, pretreatment solution, calibrators and controls should be stored between about 2 and about 8 degrees C, while the dilution buffer should be stored at ambient temperature.

It is to be appreciated that although preferred embodiments of the invention are herein described with reference to the determination of specific tricyclic antidepressants, or mixtures of tricyclic antidepressants, the concepts of the present invention can also be profitably applied not only to measurement of levels of specific ones of these drugs in various biological fluids, such as serum, plasma, spinal fluid and the like, but also to screen for the presence or amount of any of the broad category of drugs represented by the designation "tricyclic antidepressants".

## EXAMPLES

### Example 1: Preparation of N-chloroacetyl iminodibenzyl

A solution of iminodibenzyl (6.0 grams (gm)) and chloroacetyl chloride (6.0 gm) was heated at reflux in chloroform (70 milliliters ml)) for 2 hrs. After cooling to room temperature, the solution was washed with water 25 ml and 5% sodium bicarbonate (2 X 50 ml). The organic solution was dried over anhydrous magnesium sulfate, filtered and concentrated to dryness in vacuo to yield 8.3 gm of solid product.

### Example 2: Preparation of N-Aminoacetyl iminodibenzyl

A solution of N-chloroacetyl iminodibenzyl (2.25 gm) and sodium Iodide (1.85 gm) in acetone (20 ml) was stirred at room temperature for 16 hours. The mixture was diluted with chloroform (150 ml) and washed with water (4 X 25 ml). The organic solution was then dried over anhydrous magnesium sulfate, filtered and concentrated to dryness in vacuo. The residue was dissolved in methanol (50 ml), to which was added tetrabutyl ammonium bisulfate (0.1 gm) and concentrated ammonium hydroxide (50 ml). After stirring at room temperature for 68 hours, the mixture was concentrated to dryness in vacuo. The residue was then triturated with chloroform (100 ml). The organic solution was washed with water (4 X 25 ml), dried over

anhydrous magnesium sulfate and filtered. Removal of the solvent in vacuo yield 1.71 gm of crude product, which was chromatographed on silica gel (120 gm) using 10% methanol in chloroform (2 liters (L)) followed by 20% methanol in chloroform (1 L) to elute the product. The ninhydrin positive fractions were combined and concentrated to dryness in vacuo to yield 0.750 gm of product.

Example 3: Preparation of N-(2-Aminoethyl)-iminodibenzyl

To a 1 Molar (M) solution of diborane in tetrahydrofuran (15 ml) at 0°C was added N-aminoacetyliminodibenzyl (0.75 gm) in dry methylene chloride (10 ml). The solution was stirred at 0°C for 1 hour at room temperature, at reflux for 1.5 hours and at room temperature for 16 hours. 3 Normal (N) hydrochloric acid (20 ml) was then added cautiously with vigorous evolution of gas. The solution was stirred at room temperature and then concentrated to dryness in vacuo. To the residue was added water (50 ml) and sodium hydroxide until a pH of 12 was reached. The mixture was then extracted with methylene chloride (3 X 25 ml). The combined organic extracts were next washed with saturated brine (2 X 20 ml), dried over anhydrous magnesium sulfate, filtered and concentrated to a viscous oil, in vacuo. The oil was dissolved in methanol (3 ml), filtered and treated with concentrated hydrochloric acid to pH 2. The solvent was removed in vacuo. Recrystallization of the solid from chloroform/diethyl ether yielded 0.20 gm of pure product as the hydrochloride salt.

Example 4: Preparation of Immunogens

50 milligrams (mgm) of an appropriate amine was dissolved in 400 microliters (mcl) of 1 N hydrochloric acid and 400 mcl of dimethylformamide, and diluted to 2 ml with water. The solution was added to a solution of bovine serum albumin in 8 ml of water. After adjustment of the pH to 4.95 with 1 N sodium hydroxide, 52 mgm of ethyldimethylaminopropyl carbodiimide were added with stirring. After 25 minutes the pH was readjusted to 4.9 with 1 N hydrochloric acid and an additional 30 mgm of ethyl dimethylaminopropyl carbodiimide was added. After an additional 25 minutes, the solution was dialyzed against water at room temperature. Dialysis was repeated four times against 1 L of water, each time for 12 hours. This yielded an aqueous solution of purified bovine serum albumin tricyclic conjugate.

Example 5: Preparation of Tracer Compounds

A. 6-carboxyfluorescein/10,11-dihydroprotriptyline conjugate

A solution of 5 mgm of 6-carboxyfluorescein, 4.5 mgm of N-hydroxysuccinimide and 7.5 mgm of dicyclohexyl carbodiimide in 0.5 ml of dry pyridine was stirred at room temperature for 2 hours. 5 mgm of 10,11-dihydroprotriptyline hydrochloride were then added. After stirring at room temperature for 18 hours, the solution was streaked onto a silica gel thin layer chromatography plate which was developed with chloroform/methanol/acetic acid, in a ratio of 89/10/1. The appropriate band was taken and eluted with methanol. The solution was next concentrated and rechromatographed, using the above system. Collection of the appropriate band and a third chromatography on silica gel plates, using diethyl ether/methanol/acetic acid in a ratio of 95/5/0.5, yielded a substantially pure 6-carboxyfluorescein/10,11-dihydroprotriptyline conjugate.

B. 6-carboxyfluorescein/5-[B-aminoethyl]-10,11-Dihydro-5H-dibenz[b,f]azepine conjugate

A solution of 10.75 mgm of 6-carboxyfluorescein, 4.2 mgm of 1-hydroxybenztriazole and 6.45 mgm of dicyclohexylcarbodiimide in 0.4 ml of dry pyridine was stirred at room temperature for 2 hours. 6.86 mgm of 5-[B-aminoethyl]-10,11-dihydro-5H-dibenz[b,f]azepine were added. After stirring at room temperature for 18 hours, the solution was streaked onto a silica gel thin layer plate and eluted with chloroform/methanol in a ratio of 5/1. Collection of the appropriate band by extraction from the silica gel with methanol yielded 6-carboxyfluorescein/5-[B-aminoethyl]-10,11-dihydro-5H-dibenz[b,f]azepine as the pyridinium salt.

Substitution of the appropriate amino starting materials yielded the desired tracers, by utilization of substantially the previously described procedures.

## Claims

1. A compound of the formula:

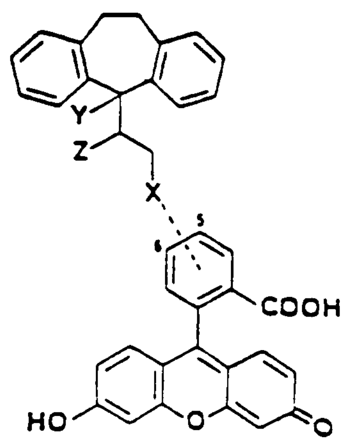

wherein Y and Z are both hydrogen or together form a bond; X is $-(CH_2)_n-NR-CO-$; n is 0 or 1 when Y and Z are hydrogen and zero when Y and Z form a bond; R is hydrogen or $CH_3$; and biologically acceptable salts thereof.

2. A fluorescence polarization immunoassay for determining the presence or amount of a tricyclic antidepressant in a fluid, which assay comprises the steps of contacting the fluid with antisera according to claim 3 or 6 to the tricyclic antidepressant and a compound of claim 1 to form a homogeneous solution, measuring the fluorescence polarization response therefrom, and determining the amount or presence of said tricyclic antidepressant in said fluid by comparing the measured fluorescence polarization response against the standard curve prepared from the polarization values obtained when measuring the fluorescence polarization responses of solutions of known concentrations of tricyclic antidepressant.

3. Antisera comprising antibody cross-reactive to a tricyclic antidepressant, wherein said tricyclic antidepressant is desipramine, amitriptyline, nortriptyline or imipramine, which antibody has been raised against an immunogen based upon a hapten of the formula:

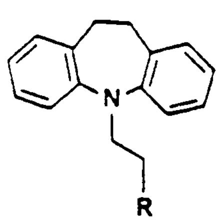

wherein R is $NH_2$ or $NHCH_3$.

4. An antibody raised against an immunogen based upon a hapten of the formula:

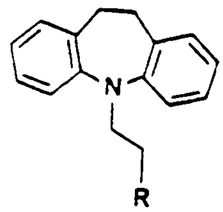

wherein R is $NH_2$ or $NHCH_3$, which antibody is cross-reactive to desipramine, amitriptyline, nortriptyline and imipramine.

5. A reagent for an immunoassay comprising antibody cross-reactive to a tricyclic antidepressant, wherein said tricyclic antidepressant is desipramine, amitriptyline, nortriptyline or imipramine, wherein said antibody has been raised against an immunogen based upon a hapten of the formula:

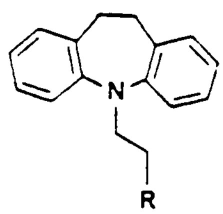

wherein R is $NH_2$ or $NHCH_3$.

6. Antisera comprising antibody cross-reactive to a tricyclic antidepressant, wherein said tricyclic antidepressant is desipramine, amitriptyline, nortriptyline or imipramine, which antibody has been raised against an immunogen based upon a hapten of the formula:

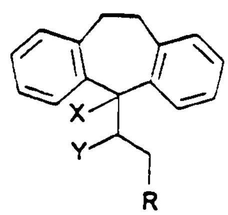

wherein R is $(CH_2)_nNH_2$ or $(CH_2)_nNHCH_3$; and X and Y are both hydrogen or together form a bond; and n is 0 or 1 when X and Y are hydrogen, and zero when X and Y form a bond.

**7.** An antibody raised against an immunogen based upon a hapten of the formula

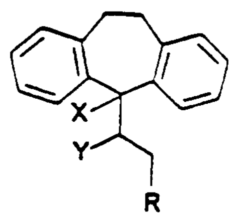

wherein R is $(CH_2)_nNH_2$ or $(CH_2)_nNHCH_3$; and X and Y are both hydrogen or together form a bond; and n is 0 or 1 when X and Y are hydrogen, and zero when X and Y form a bond, which antibody is cross-reactive to desipramine, amitriptyline, nortriptyline and imipramine.

**8.** A reagent, for an immunoassay, comprising antibody cross-reactive to a tricyclic antidepressant, wherein said tricyclic antidepressant is desipramine, amitriptyline, nortriptyline or imipramine, wherein said antibody has been raised against an immunogen based upon a hapten of the formula

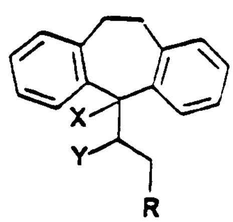

wherein R is $(CH_2)_nNH_2$ or $(CH_2)_nNHCH_3$; X and Y are both hydrogen or together form a bond; and n is O or 1 when X and Y are hydrogen, and zero when X and Y form a bond.

**Patentansprüche**

**1.** Eine Verbindung der Formel:

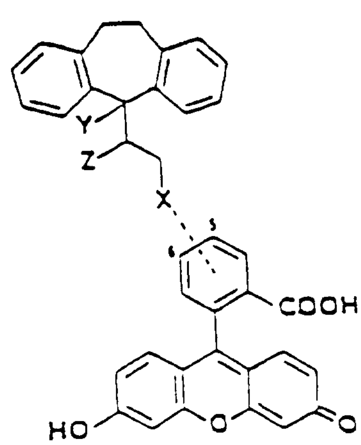

wobei Y und Z beide ein Wasserstoffatom sind oder zusammen eine Bindung bilden; X ist $-(CH_2)_n$-NR-CO-; n ist 0 oder 1, wenn Y und Z ein Wasserstoffatom sind, und n ist 0, wenn Y und Z eine Bindung bilden; R ist Wasserstoff oder $CH_3$; und biologisch verträgliche Salze dieser Verbindungen.

2. Ein Immunoassay, beruhend auf Fluoreszenzpolarisation, um die Gegenwart oder die Menge eines tricyclischen Antidepressivums in einer Flüssigkeit zu bestimmen, wobei dieser Assay die folgenden Schritte umfaßt: das Mischen von Flüssigkeit, welche Antiseren gemäß den Ansprüchen 3 oder 6 bezüglich dem tricyclischen Antidepressivum enthält und einer Verbindung entsprechend Anspruch 1, um eine homogene Lösung zu bilden, die Messung des Fluoreszenzpolarisationssignals von dieser und die Bestimmung der Menge oder der Anwesenheit des tricyclischen Antidepressivums in der Flüssigkeit durch Vergleich mit der Eichkurve, erhalten aus den Polarisationswerten der Messung des Fluoreszenzpolarisationssignals von Lösungen bekannter Konzentration an tricyclischem Antidepressivum.

3. Ein Antiserum, beinhaltend einen Antikörper welcher kreuzreaktiv zu einem tricyclischen Antidepressivum ist, wobei das tricyclische Antidepressivum Desipramin, Amitriptylin, Nortriptylin oder Imipramin ist, und wobei der Antikörper entgegen einem Immunogen, basierend auf dem Hapten der Formel

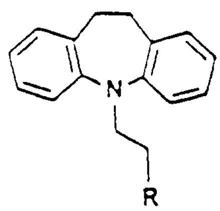

wobei R gleich $NH_2$ oder $NHCH_3$ ist, gebildet wurde.

4. Ein Antikörper, gebildet gegen ein Immunogen, welches auf einem Hapten folgender Formel aufbaut:

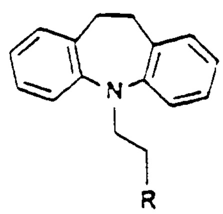

wobei R gleich $NH_2$ oder $NHCH_3$ ist, und wobei der Antikörper kreuzreaktiv zu Desipramin, Amitryptilin, Nortriptylin und Imipramin ist.

5. Ein Reagens für ein Immunoassay, welches einen Antikörper beinhaltet, der kreuzreaktiv zu einem tricyclischen Antidepressivum ist, wobei das tricyclische Antidepressivum Desipramin, Amitriptylin, Nortripytilin oder Imipramin ist, wobei der Antikörper entgegen einem Immunogen basierend auf einem Hapten der Formel

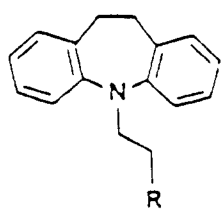

wobei R gleich $NH_2$ oder $NHCH_3$ ist, gebildet wurde.

**6.** Ein Antiserum, einen Antikörper umfassend, welcher kreuzreaktiv zu einem tricyclischen Antidepressivum ist, wobei das tricyclische Antidepressivum Amitriptylin, Nortriptylin oder Imipramin ist, wobei der Antikörper entgegen einem Immunogen basierend auf einem Hapten der Formel gebildet wurde:

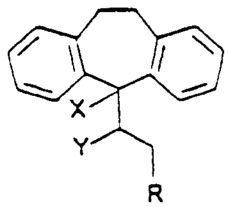

wobei R gleich $(CH_2)_nNH_2$ oder $(CH_2)_nNHCH_3$ ist, und X und Y sind beide Waserstoffatome oder bilden zusammen eine Bindung, und n ist 0 oder 1, wenn X und Y Wasserstoffatome sind, und n ist 0, wenn X und Y eine Bindung bilden.

**7.** Ein Antikörper, gebildet entgegen einem Immunogen, basierend auf einem Hapten der Formel

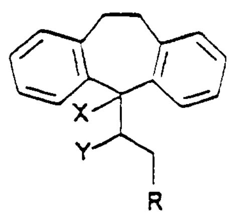

wobei R gleich $(CH_2)_nNH_2$ oder $(CH_2)_nNHCH_3$ ist, und X und Y sind beide Wasserstoffatome oder bilden eine Bindung, und n ist 0 oder 1 wenn X und Y Wasserstoff sind, und n ist 0, wenn X und Y eine Bindung bilden , wobei der Antikörper kreuzreaktiv zu Desipramin, Amitryptilin, Nortryptilin und Imipramin ist.

**8.** Ein Reagens für einen immunologischen Bestimmungstest, beinhaltend einen Antikörper, der zu einem tricyclischen Antidepressivum kreuzreaktiv ist, wobei das tricyclische Antidepressivum Desipramin, Amitryptilin, Nortryptilin oder Imipramin ist, und wobei der Antikörper gebildet wurde gegen ein Immunogen basierend auf auf einem Hapten der Formel

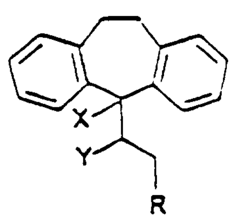

wobei R gleich $(CH_2)_nNH_2$ oder $(CH_2)_nNHCH_3$ ist, X und Y beide Wasserstoffatome sind oder zusammen eine Bindung bilden, und n ist 0 oder 1, wenn X und Y Wasserstoffatome sind und n ist 0, wenn X und Y eine Bindung bilden.

## Revendications

1. Composé de formule:

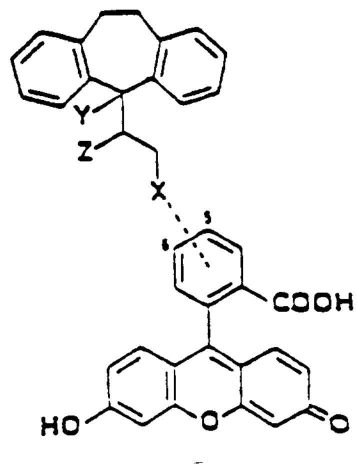

dans laquelle Y et Z sont tous les deux hydrogène ou forment ensemble une liaison ; X est $-(CH_2)_n$-NR-CO- ; n est 0 ou 1 lorsque Y et Z sont hydrogène et zéro lorsque Y et Z forment une liaison ; R est hydrogène ou $CH_3$ ; et des sels biologiquement acceptables de ceux-ci.

2. Immunotest de polarisation avec fluorescence pour déterminer la présence ou la quantité d'un antidépresseur tricyclique dans un liquide, lequel test comprend les étapes de mise en contact du liquide avec des antisérums selon les revendications 3 ou 6 avec l'antidépresseur tricyclique et un composé de la revendication 1 pour former une solution homogène, pour en mesurer la polarisation avec fluorescence , et déterminer la quantité ou la présence dudit antidépresseur tricyclique dans ledit liquide en comparant la réponse de polarisation avec fluorescence mesurée à la courbe standard préparée à partir des valeurs de polarisation obtenues en mesurant les réponses de polarisation avec fluorescence de solutions de concentrations connues d'antidépresseurs tricycliques.

3. Antisérums comprenant une réactivité de liaison d'un anticorps à un antidépresseur tricyclique, dans lequel ledit antidépresseur tricyclique est desipramine, amitriptyline, nortriptyline ou imipramine, lequel anticorps est obtenu à partir d'un immunogène à base d'un haptène de formule :

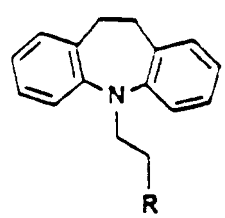

dans laquelle R est $NH_2$ ou $NHCH_3$.

**4.** Anticorps obtenu à partir d'un immunogène à base d'un haptène de formule :

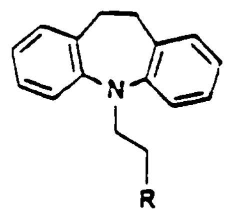

dans laquelle R est $NH_2$ ou $NHCH_3$, lequel anticorps est un anticorps à réactivité de liaison avec la desipramine, l'amitriptyline, la nortriptyline et l'imipramine.

**5.** Réactif pour un immunotest comprenant un anticorps à réactivité de liaison à un antidépresseur, dans lequel ledit antidépresseur tricyclique est desipramine, amitriptyline, nortriptyline ou imipramine, dans lequel ledit anticorps a été obtenu à partir d'un immunogène à base d'un haptène de formule:

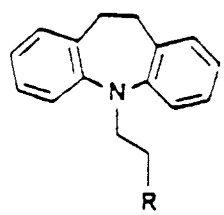

dans laquelle R est $NH_2$ ou $NHCH_3$.

**6.** Antisérums comprenant une réactivité de liaison d'un anticorps à un antidépresseur tricyclique, dans lequel ledit antidépresseur tricyclique est desipramine, amitriptyline, nortriptyline ou imipramine, lequel anticorps a été obtenu à partir d'un imunogène à base d'un haptène de formule :

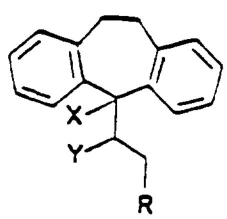

dans laquelle R est $(CH_2)_nNH_2$ ou $(CH_2)_nNHCH_3$ ; et X et Y sont tous les deux hydrogène ou forment ensemble une liaison, et n est 0 ou 1 lorsque X et Y sont hydrogène, et zéro lorsque X et Y forment une liaison.

**7.** Anticorps obtenu à partir d'un immunogène à base d'un haptène de formule :

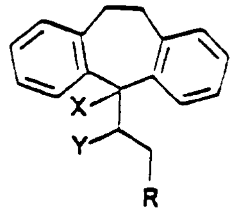

dans laquelle R est $(CH_2)_nNH_2$ ou $(CH_2)_nNHCH_3$ ; et X et Y sont tous les deux hydrogène ou forment ensemble une liaison, et n est 0 ou 1 lorsque X et Y sont hydrogène, et zéro lorsque X et Y forment une liaison, lequel anticorps a une réactivité de liaison à la desipramine, l'amitriptyline, la nortriptyline et l'imipramine.

**8.** Réactif, pour un immunotest, comprenant un anticorps à réactivité de liaison à un antidépresseur tricyclique, dans lequel ledit antidépresseur est desipramine, amitriptyline, nortriptyline ou imipramine, dans lequel ledit anticorps est obtenu à partir d'un immunogène à base d'un haptène de formule:

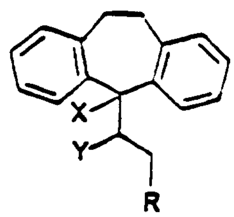

dans laquelle R est $(CH_2)_nNH_2$ ou $(CH_2)_nNHCH_3$ ; et X et Y sont tous les deux hydrogène ou forment ensemble une liaison, et n est 0 ou 1 lorsque X et Y sont hydrogène, et zéro lorsque X et Y forment une liaison.